Europäisches Patentamt

(19) European Patent Office

Office européen des brevets

(11) Publication number: **0 263 796**
**A1**

# EUROPEAN PATENT APPLICATION

(21) Application number: **87830349.4**

(22) Date of filing: **05.10.87**

(51) Int. Cl.⁴: **C 02 F 3/28**

(30) Priority: **08.10.86 IT 4852586**

(43) Date of publication of application:
**13.04.88 Bulletin 88/15**

(84) Designated Contracting States: **BE DE ES FR GR**

(71) Applicant: **ENEA COMITATO NAZIONALE PER LA RICERCA E PER LO SVILUPPO DEL L'ENERGIA NUCLEARE E DELLE ENERGIE ALTERNATIVE**
**Viale Regina Margherita 125**
**I-00198 Roma (IT)**

(72) Inventor: **De Poli, Fabrizio**
**Via Montebello 22**
**I-00185 Roma (IT)**

**Tilche, Andrea**
**Via Enrico Fermi 11**
**I-40033 Casalecchio di Reno (IT)**

**Tesini, Oriano**
**Via Togliatti 22 San Matteo della Decima**
**I-40040 San Giacomo Persiceto (IT)**

(74) Representative: **Sarpi, Maurizio**
**Studio FERRARIO Via Collina, 36**
**I-00187 Roma (IT)**

(54) **Process and device for producing a biological gas from the anaerobical digestion of an organic substance.**

(57) In a two-phase (acid and methane phases) process for producing biogas the substrate to be treated is fed to the first reactor space (1), where it is heated by a heat exchanger (2) to a temperature above 37°C. In the second reactor space a heat exchanger (6) holds the temperature under 37°C. The temperature gradient ensures the flow between the two spaces without stirring.

FIG. 1

**Description**

PROCESS AND DEVICE FOR PRODUCING A BIOLOGICAL GAS FROM THE ANAEROBICAL DIGESTION OF AN ORGANIC SUBSTANCE.

It is known, that the transformation in an anaerobical surrounding of the organic substance comprises the forming of a biogas (a mixture of methane and carbon dioxide) by a population comprised of microorganisms; such process is already widely adopted and used also in the industry both for producing energetic products and for the environment protection from the point of view of the reduction of the organic tainting charge and of the reduction of the unpleasant smells.

It is known that the digestion anaerobic process comprises several biochemical reactions which can be grouped in two phases: a first hydrolysis phase and production of volatile acid fats due to a microorganisms flora also microaerophilic the activity of which is positively influenced by a temperature up to 55-65°C and which evolves also at a pH which is lower than the neutrality; a second methane production phase starting from volatile fat acids and starting from the synthesis of hydrogen and carbondioxide due to particular microorganisms which are called methanogens and are strictly anaerobes, their pH limits being comprised between the neutrality and 8.5 pH the optimum temperatures being 35°C for the mesophile process and 55°C for the thermophile process.

The reactors for producing the biogas which are defined anaerobic digesters have been widely diffused both nationally and internationally and adopt several technologies; in nearly the total of the cases the two phases in such reactors operate with no separation at all.

The experiments conducted in several international research centers have shown how the process efficiency, above all in the presence of an organic substance with a total solid content greater than 8% could be raised by separating the two phases whereby the same can evolve in such conditions which are particularly favourable to the respective requirements; specifically it has been shown that a first phase at a temperature higher than 35°C, which is normally used in the mesophile process a more rapid hydrolysis of the organic substance with particular reference to the cellulosic fibres; as far as the second phase the rising up of the temperature beyond the above limits has the effect of not improving the conversion into methane while increasing the energy consumption for the maintenance of the process temperatures. The realisation of two separate reactors for the two phases implies not acceptable increases of the plant cost. For this reason, up to now, plants of this type have not been realized in actual scale of commercial caracter.

A particular reference is to be made to the mixing method of the organic substance; in the conventional reactors in order to allow a thermal homogeneity and a contact between the substrate and the bacterial population, an agitation system is necessary which could be mechanical, hydraulical or by gas insufflation and which comprises a notable increase of the realisation and operation costs such as the energy consumption and the maintenance; for those substrates which have a high organic substance content (ST 8%) such costs become still higher, due to the greater density of the substrate and to the frequent presence of coarse material.

It is known that in order to obviate such inconveniencies, "plug-flow" plants have been designed which are comprised of usually horizontal reactors wherein the organic substance which already contained at the origin the microbic flora, is displaced without any mixing while reaching the process temperature due to a static heating system, with heat diffusion consequent to the convective motions and to the migration of the originated gas bubbles; such plants, which are characterized by acceptable realisation costs, did not give the hoped results because the lack of an initial hydrolysis phase implied the formation of fibrous material crusts with consequent block of the reactor.

The purpose of this invention is to unify the two-stage process (and the consequent greater conversion effectiveness) with the plug-flow process which is characterized by low construction costs thereby obtaining that the two phases of the organic substance to be treated that is the hydrolysis acidogenesis and methanegenesis phase, evolve in succession and with no stirring in two different areas of a single reactor divided by a separating element suitably dimensioned and positioned; in the first area the temperature is maintained at values higher than 37°C at the purpose of favouring the hydrolysis and acidogenesis processes while in the second area the temperature is lower than 37°C with the purpose of favouring the methanegenesis phase.

Such temperature difference is obtained by a suitable insuring thermal gradient in the two areas and by using the separator element also to the purpose of creating a preferential path of the substrate between said areas through the convective motions of the same substrate.

In order to actuate the process according to the invention a reactor is provided wherein the organic substance to be treated is admitted and flows which reactor comprises a single compartment or container by which a piston flow is warranted which is characterized by a high ratio (3-6) between the length and the cross section of the piston, a separator element being present in such container which divides it into two areas which communicate with one another; a heating element being inserted in the first area such that the temperature of the admitted fluid is brought above 37°C and the passage of the biomass into the second area through convective motion is favoured. This second area has no heater or it has heating elements adapted for maintaining the temperature at a value lower than 37°C.

Because the mass flow is ensured in a continous way from the first to the second area, the loading occurs at the inside of the first area of the reactor by means of a pump or by gravity while the effluent exits from the second area.

The reactor thus realized is a consisting improvement with respect to what has been fabricated up to now in as much as it allows conversion indexes which are 40% higher in the biogas production from which cow dejections and a reduction of the construction costs worthy about 75/80% with respect to conventional plants.

As for the reactor heating system this is static and composed by pipes or belts with a circulation of heating fluid at a temperature not higher than 70°C in order to avoid any adhesion of organic substance to the walls of the heating system; such exchanger can be made either by metal or by plastic material. The latter solution implies a lower realisation cost and a larger resistance to corrosion and does not reduce consistently the thermal exchange because the greater limit is represented by the reduced value of the exchange between the outer surface of the exchanger and the non moving substrate which makes very low in percentage the thermal resistance due to the material by which the exchanger is made.

The reactors by which the process of the invention is used are typically horizontal; however the flow can also be vertical for substrates having high presences of inert substances.

The description of the invention will be better understood and illustrated by referring to the attached drawings which represent as a non limitative examples some preferred realisation forms.

In the drawings:

Fig. 1 is a cross section along to line A-A of fig. 2 of a reactor with a circular plan view having a first central room and a concentric crown as a second room.

Fig. 2 is a plan view starting form line B-B of fig. 1.

Fig. 3 is a plan view of an alternative form of a reactor according to the invention having a U plan view.

Fig. 4 and 5 show two vertical cross sections respectively according to lines IV-IV and V-V of Fig. 3.

Fig. 6 and 7 are respectively a vertical cross section and a plan view of a further variant of a reactor with rectangular plan view.

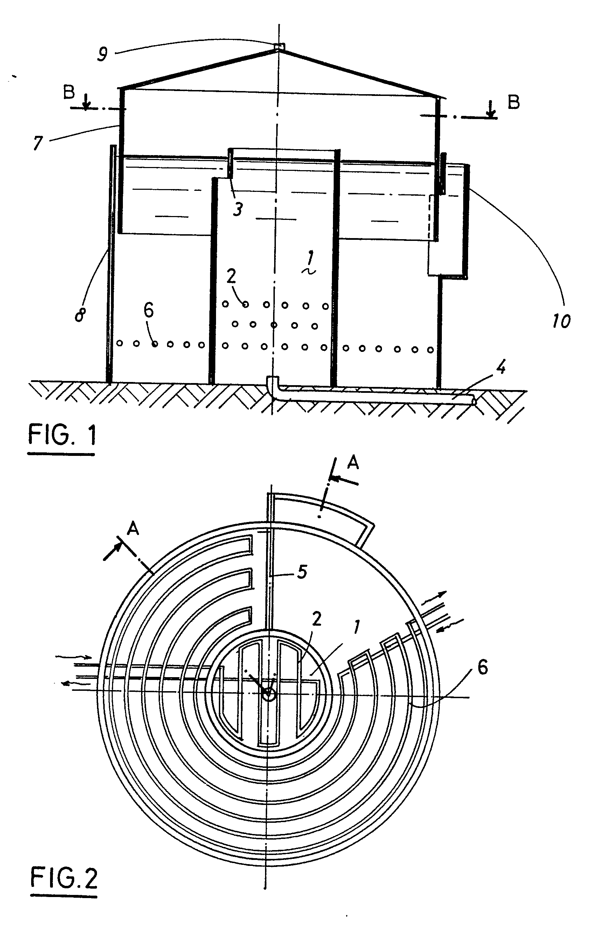

With reference to figures 1 and 2 in the horizontal configuration, the reactor comprises a first room 1 wherein the substrate to be treated, introduced at ambient temperature through conduit 4, is heated at temperature 40-45°C in relatively rapid times through an exchange comprising a serpentine 2 made of tubes or by a heating jacket through which a fluid circulates at a temperature not above 70°C; due to the density difference caused by the different temperatures, the incoming substrate tends to stay at the lower zone of the room and is then heated at a temperature which is an optimum for the processes of the first phase of the anaerobic digestion; only later, due to the variation of temperature and density, the material moves to the higher part of the first room and is consequently adapted for overcoming the separator element 3 by which the first section is divided from the successive one; in the second section of the reactor occurs the very production of a biogas; in this section another wall 5 can be inserted with the function of crust breaker with an opening located downwards. In the second section a static heating maintenance 6 system is usually inserted for consenting to the reactor of working at the optimum temperature (usually 35°C).

The two walls and the heating system of the second section may not be strictly necessary in the case when the substrate does not lend itself to stratifications and the hydraulic residing time is enough short so as to avoid an excessive cooling of the substrate.

As far as the upper part of the plant is concerned a gaslight cover 7 is provided which is made either by flexible materials such as rubber, plastic material etc. or by rigid material such as resin-glass, metal etc. On the cover the pipe 9 for collecting the gas is engaged; said cover should be readily removable in order to permit any interventions into the inside of the plant and constitutes then an innovation with respect to simplified plants as conventionally used in Emergent Countries which plants have a fixed cover and cannot then be opened for maintainance; the anchorage of cover 7 at the inside of a liquid 8 head which may be water in a suitable channel or the same substrate with an under level hook comprises a novelty by which any gas loss can be advantageously suppressed and at the same time comprises a safe hydraulic guard against any overpressure. Other sealing methods can also be adopted of the dry type but in this case safety valves are necessarily applied.

When the methanegenesis process is terminated the effluent gets out through a suitable collector 10. The use of insulation both on the walls and on the cover depends on the climate conditions and on the evaluation of the economical advantages due to the lower self-consumption of biogas for heating the plant; the latter can be both laid underground or uncovered taking mainly into account the heat losses and the related insulation.

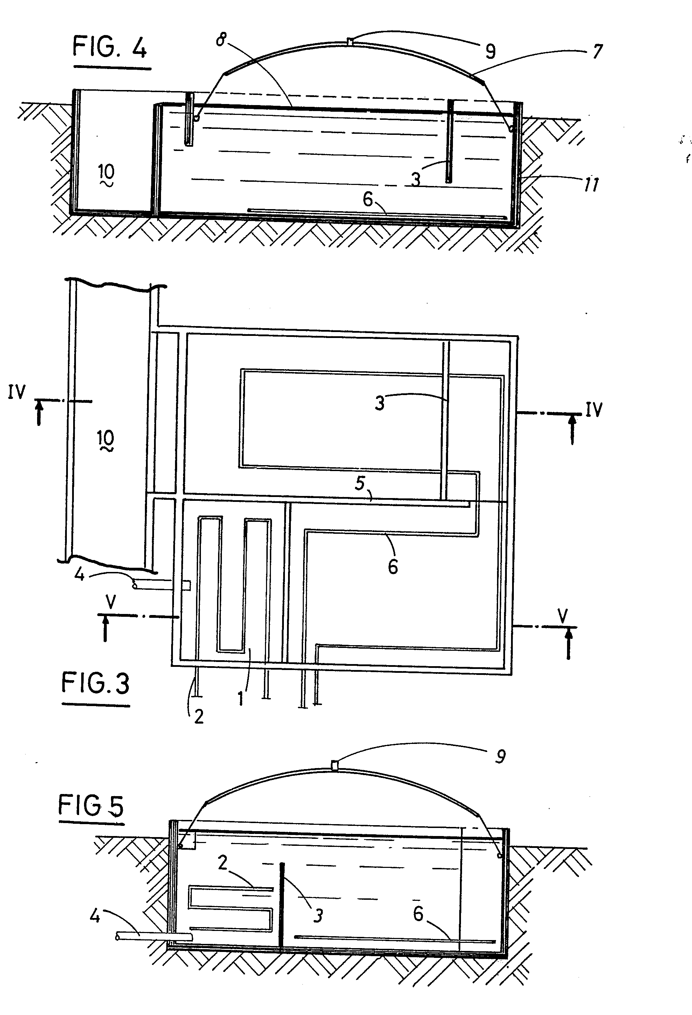

In Fig. 3, 4 and 5 a variant is shown of the plant as above described. This is the case of a "U" reactor, such being the shape of the trajectory followed by the mass of organic substance during its treatment.

Conduit 4 for feeding the biomass flows into the treatment first room 1 which has a substantially rectangular form. In this room the influent is heated due to coil 2 so that the first phase of the process is carried out, while the second room, which as already told has the shape of an "U" is defined by wall 3 which is interrupted between the two zone and is assigned to effecting the methanization phase during which any secondary thermal treatment is trusted to coil 6. The removable cover 7 is airtight along its edges and is provided at its vertex with the collecting pipe 9 of the methane. The effluent falls into the outlet collector 10. This variant the same as in the already described solution, consents a reduction of the surface of exchange with the outside, with the consequent reduction of the heat losses and of the insulation costs.

**0 263 796**

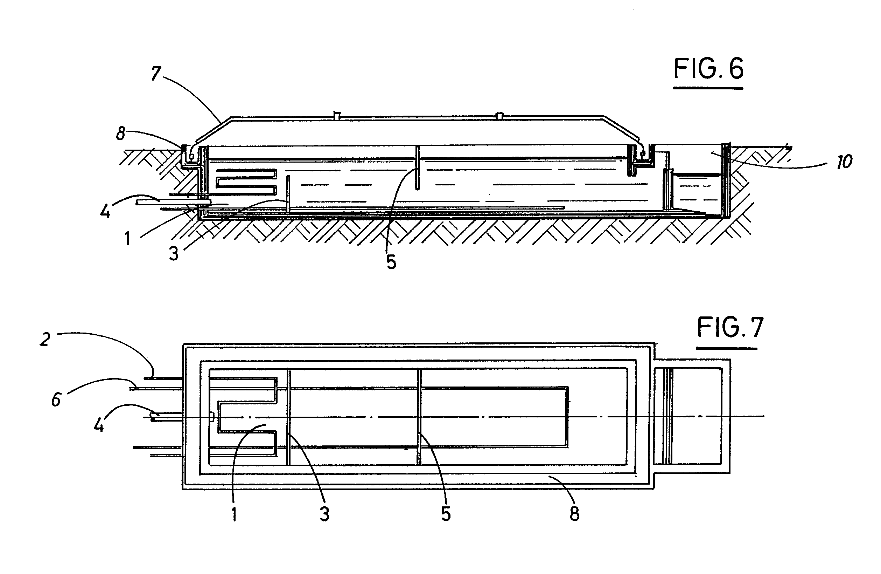

A further variant is shown in figures 6 and 7. In this case the two treatment rooms are in continuation therebetween, whereby the biomass follows substantially 2 rectilinear path; it enters room 1 and undergoes a first thermal treatment due to coil 2 and thereafter it overcomes the divisional transversal wall 3 and gets into the second room wherein secondary heating coil 6 is located along with the breaking- crust 5 which is located at a certain height with respect to the tank bottom as shown hereinbefore.

The removable cover 7 in this case has at its base a peripheral water head or channel which displays the double function of misuring the seal and of avoiding any overpressures. The biogas produced is picked up by one or more collecting pipes 9 while the effluent mass falls down in the outlet collector 10 as in the proceding cases.

The present invention has been illustrated and described with reference to some preferred fulfillments but it is understood that construction variants can be substantivally introduced into said fulfillments without getting out of the scope of this invention.

## Claims

1. A double phase process for producing a biogas characterized by the fact that the two phases, respectively hydrolysis-acidogenesis and methanogenesis of the organic substance to be treated evolve in succession and without any stirring in two areas of a single reactor, in the first area the temperature of the mass being greater than 37°C, while in the second area is lower than 37°C, such temperature difference being obtained by ensuring in the two areas an adequate thermal gradient and by utilizing a separator element tending to favour convective motions of the substrate and to create a preferential path of the same substrate between the two areas.

2. A process according to claim 1 characterized by the fact that the temperatures of the two areas are obtained by means of stationary heating elements comprised of coils which are passed through by a fluid at a temperature lower than 70°C.

3. A plant for the production of biogas by means of the process according to claims 1 and 2 characterized by the fact of comprising a single room or container wherein the organic substance to be treated is put in and flows, said container being provided with a separator element which divides it into two areas communicating with one another of which the first is provided with an heating element of which the function is for taking the temperature of the organic mass above 37°C and for favouring through convective motions the passage of the same mass into the second area wherein no heating element is provided or a heating element is provided adapted for maintaining the temperature below 37°C.

4. A plant according to claim 3, characterized by the fact that the feeding of the first area is obtained by means of a pump or by gravity in such a way that a continous thrust is created whereby the flux of the organic mass is determined from the first to the second area and the outlet from the latter of the mass which underwent the treatment.

5. A plant according to claims 3 and 4 characterized by the fact that at least the methanization area is provided with a cover of a gastight material, readily removable and provided with means for capturing and sending the same gas to a system for accumulating, distributing or directly utilizing it.

6. A plant according to claims 3-5 characterized by the fact that said cover is provided with a peripheral sealed closure, located in a liquid head or channel.

7. A plant according to claims 3-6 characterized by the fact that in the second treatment area a crust-breaking wall is provided which is located transversally with respect to the direction of the bio-mass flux and lifted with respect to the tank bottom.

7. A plant according to claims 3-7 characterized by the fact that the reactor has a cylindrical form and the two areas are disposed in concentric position with the first area at the center.

9. A plant as claimed in claims 3-7 characterized by the fact that the two areas are disposed in such a way that the mass fulfills a U travel,

10. A plant according to claims 3-7 characterized by the fact that the two areas are disposed in continuation to one another.

11. A process for producing a biogas by means a double stage in a single reactor, and a plant for utilizing such process according to claims 1-10 substantially as described and illustrated.

0263796

FIG. 1

FIG.2

0263796

FIG. 4

FIG. 3

FIG 5

FIG.6

FIG.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 158 213 (L. KIRMAIR)<br>* Page 38, claims 1,3,19,26; page 29, line 31 - page 31, line 1 *<br>--- | 1-6,7-10 | C 02 F 3/28 |
| X | NL-A-8 001 072 (DE ERVEN G. DE BOER B.V.)<br>* Page 8, claims 1,3,6,7,9; figures 1,2 *<br>--- | 1-5,7-10 | |
| X | EP-A-0 120 772 (BERTIN & CIE.)<br>* Page 9, claims 1-5 *<br>--- | 1-5,10 | |
| X | EP-A-0 145 792 (BIORGANIC ENERGY)<br>* Page 14, claims 1,3; page 4, line 29 - page 5, line 30; page 8, lines 16-33 *<br>--- | 1-5,7,9,10 | |
| A | FR-A-2 376 209 (A. MAUMONT et al.)<br>* Page 3, claim 1; page 1, lines 27-33; figure 1 *<br>--- | 5,6 | |
| A | KORRESPONDENZ ABWASSER, vol. 33, October 1986, pages 877-893, GFA, St. Augustin, (DE); C.F. SEYFRIED et al.: "Verfahren der anaeroben Reinigung von Industrieabwässern"<br>* Page 878, right-hand column, lines 6-17 *<br>----- | | TECHNICAL FIELDS SEARCHED (Int. Cl.4)<br><br>C 02 F<br>C 12 M |

The present search report has been drawn up for all claims

| Place of search | Date of completion of the search | Examiner |
|---|---|---|
| THE HAGUE | 12-01-1988 | TEPLY J. |

EPO FORM 1503 03.82 (P0401)